# EUROPEAN PATENT APPLICATION

(11) **EP 2 832 372 A1**
(43) Date of publication of application: **04.02.2015**
(21) Application number: 12872816.9
(22) Date of filing: 26.03.2012
(51) Int. Cl.: A61K 47/38, A61K 47/30, A61K 9/48, A61K 9/50

(54) **COMPOSITION FOR ENTERIC HARD CAPSULE AND ENTERIC HARD CAPSULE PREPARED USING THE COMPOSITION**

(71) Applicant: Samsung Fine Chemicals Co., Ltd., Ulsan-city 680-090 (KR)
(72) Inventor: SON, Jin Ryul, Incheon 403-020 (KR); PARK, Eun Hee, Incheon 405-220 (KR); BAEK, Hyon Ho, Incheon 406-130 (KR)
(74) Representative: Kador & Partner
(86) International application number: PCT/KR2012/002192
(87) International publication number: WO 2013/147329

(57) **Abstract**

A composition for enteric hard capsules and an enteric hard capsule prepared using the composition. The composition includes a water-soluble divalent base and an alkaline agent. The enteric hard capsule has high transparency and delaying effects of separation of salt during storage.

## Description

### TECHNICAL FIELD

The present invention relates to a composition for enteric hard capsules, and an enteric hard capsule prepared using the composition, and more particularly, to a composition for enteric hard capsules that includes a water-soluble divalent base and an alkaline agent, and an enteric hard capsule prepared using the composition.

### BACKGROUND ART

Capsules for pharmaceutical preparations and nutraceutical preparations are generally prepared using gelatin, hydroxypropyl methylcellulose (HPMC) or hydroxypropyl methylcellulose phthalate (HPMCP) as base materials.

Gelatin capsules have high industrial productivity and high price competitiveness. However, if gelatin capsules contain 10 wt% moisture or less, they may lose plasticity and may exhibit dramatic deterioration in impact resistance. In addition, a concern on the mad cow disease has limited the use of gelatin capsules. Therefore, plant-based HPMC capsules and enteric HPMCP capsules prepared without gelatin have drawn attention.

However, conventional HPMCP capsules have low transparency and are fragile at a low moisture content of 10 wt% or less, for example. In addition, when being stored in severe high-temperature conditions, the conventional HPMCP capsules may lose interior moisture slowly and harden because they include an excess amount of a neutralizing agent (e.g., alkaline agent). Furthermore, the neutralizing agent may gradually separate from the hardened capsules, which is also called a separation of salt. The separation of salt does not occur for 12 months or longer when the HPMCP capsules are stored in a hermetically sealed package, while the separation of salt occurs within approximately 12 months when the HPMCP capsules are stored in a generally packaged form or an unpackaged form.

### DETAILED DESCRIPTION OF THE INVENTION

### TECHNICAL PROBLEM

The present invention provides a composition for enteric hard capsules that includes a water-soluble divalent base and an alkaline agent.

The present invention also provides an enteric hard capsule prepared using the composition.

### TECHNICAL SOLUTION

According to an aspect of the present invention, there is provided a composition for enteric hard capsules, the composition including: 50 to 90 parts by weight of hydroxypropyl methylcellulose phthalate (HPMCP); 10 to 50 parts by weight of hydroxypropyl methylcellulose (HPMC); 0.1 to 2.5 parts by weight of a water-soluble divalent base; and 3.5 to 7.5 parts by weight of an alkaline agent, wherein the amounts of the HPMCP, the HPMC, the water-soluble divalent base, and the alkaline agent in parts by weight are based on 100 parts by weight of the total weight of the HPMCP and the HPMC.

The HPMCP may include 20 to 24 wt% of a methoxy group, 6 to 10 wt% of a hydroxypropoxy group, and 21 to 26 wt% of a phthalyl group, and may have a kinetic viscosity of 48 cSt to 66 cSt.

The water-soluble divalent base may be calcium hydroxide.

The alkaline agent may be ammonia, sodium hydroxide, potassium hydroxide, or a combination thereof.

The composition may further include 1 to 15 parts by weight of a plasticizer based on 100 parts by weight of the total weight of the HPMCP and the HPMC.

The plasticizer may include hydrogenated corn syrup, triethyl citrate (TEC), triacetin (TA), polyethylene glycol (PEG), propylene glycol (PG), or a combination thereof.

The composition may further include 0.05 to 0.5 parts by weight of an emulsifier based on 100 parts by weight of the total weight of the HPMCP and the HPMC.

The emulsifier may include one selected from the group consisting of sodium lauryl sulfate (SLS), sugar ester (SE), and combinations thereof.

The compound may further include water, wherein a ratio (Ww/Wm) of the amount of water (Ww) to the total amount of the HPMCP and the HPMC (Wm) may be in a range of 75 to 85 parts by weight/15 to 25 parts by weight.

According to another aspect of the present invention, there is provided an enteric hard capsule prepared using the composition described above.

### ADVANTAGEOUS EFFECTS

According to the one or more embodiments of the present invention, there is provided a composition for enteric hard capsules that includes a water-soluble divalent base and an alkali agent.

According to the one or more embodiments of the present invention, an enteric hard capsule prepared by using the composition may have high mechanical strength (e.g., impact resistance) and transparency, and separation of salt from the enteric hard capsule may be delayed during storage.

### BEST MODE

Hereinafter, exemplary embodiments of the present invention will be described in detail.

According to an embodiment of the present invention, a composition for enteric hard capsules may include: 50 to 90 parts by weight (for example, 60 to 80 parts by weight) of hydroxypropyl methylcellulose phthalate (HPMCP); 10 to 50 parts by weight (for example, 20 to 40 parts by weight) of hydroxypropyl methylcellulose (HPMC); 0.1 to 2.5 parts by weight (for example, 1.0 to 1.5 parts by weight) of a water-soluble divalent base; and 3.5 to 7.5 parts by weight (for example, 4.5 to 5.5 parts by weight) of an alkaline agent, wherein the amounts of the HPMCP, the HPMC, the water-soluble divalent base, and the alkaline agent in parts by weight are based on 100 parts by weight of the total weight of the HPMCP and the HPMC.

The HPMCP is not disintegrated at a pH of gastric juice (pH of about 1.2) for 2 to 4 hours or longer, and is rapidly disintegrated at a pH of small intestinal juice (pH of about 6.8) within 10 minutes. The HPMCP may include, for example, 20 wt% to 24 wt% of a methoxy group, 6 wt% to 10 wt% of a hydroxypropoxy group, and 21 wt% to 26 wt% of a phthalyl group, and may have a kinetic viscosity of 48 cSt to 66 cSt. The amounts of the methoxy group, the hydroxypropoxy group, and the phthalyl group in weight percentage are based on the total weight of the HPMCP. Throughout the specification, the terms "kinetic viscosity" and "viscosity" indicate the viscosity measured using an Anton Paar MCR 301 (heating rate: 2□/min, Spindle No.: CC 27 8009, RPM (shear rate): 1/s; available from Anton Paar), and specifically, the term "kinetic viscosity of the HPMCP" indicates the viscosity of 20 wt% aqueous solution of the HPMCP measured as described above. An enteric hard capsule may be prepared by using the composition for enteric hard capsules that contains HPMCP having these physical characteristics, whereby the enteric hard capsule may have good film strength, good transparency, and good elasticity. In addition, when an enteric hard capsule is prepared by using the composition including the HPMCP, a less amount of the alkaline agent may be used. This may delay the separation of salt, which indicates that alkaline components, for example, Na⁺, K⁺, and Ca²⁺ are separated from capsule films during the storage of the enteric hard capsules. However, embodiments of the present invention are not limited thereto. For example, the HPMCP may have different physical properties from those of the HPMCP listed above. When the amount of the HPMCP is within the range of 50 to 90 parts by weight based on 100 parts by the total weight of the HPMCP and the HPMC, the aqueous composition may have a viscosity that is appropriate to form capsules with an appropriate thickness, and capsules formed from the aqueous composition may have good enteric characteristics.

The HPMC may improve elasticity of the fragile enteric capsule film and enteric capsule formability, and may be used to adjust a gelation start temperature of the composition to a temperature range of, for example, 20°C to 70°C, which is applicable in commercial production. The HPMC may include 4 wt% to 12 wt%, for example, 4 wt% to 7.5 wt%, of a hydroxypropoxy group and 19 wt% to 30 wt%, for example, 27 wt% to 30 wt%, of a methoxy group. The amounts of the hydroxypropoxy group and the methoxy group in weight percentage are based on the total weight of the HPMC, respectively. The viscosity of 2 wt% aqueous solution of the HPMC may be from 3 cps (centipoise) to 50 cps, for example, from 3 cps to 15 cps. When the amount of the HPMC is within the range described above (i.e., 10 parts by weight to 50 parts by weight), the capsule formability may be good, and the prepared capsules may have good elasticity and good enteric characteristics.

The water-soluble divalent base may further delay the separation of salt. In addition, by using the water-soluble divalent base, less amounts of the alkaline agent and a plasticizer, which will be described below, may be used. The water-soluble divalent base may include calcium hydroxide. When the amount of the water-soluble divalent base is within the range of 0.1 to 2.5 parts by weight based on 100 parts by the total weight of the HPMCP and the HPMC, the water-soluble divalent base may exhibit the same properties as those of the alkaline agent, improve transparency of the prepared enteric hard capsule which lead to good appearance of the enteric hard capsule, and effectively delay the separation of salt.

The alkaline agent is an alkaline agent other than the water-soluble divalent base, and may solubilize the HPMCP. The alkaline agent may be a basic material such as ammonia, sodium hydroxide, potassium hydroxide, or a combination thereof. The alkaline agent may affect the gelation start temperature. As used herein, the term "gelation start temperature" indicates the temperature at which the viscosity of the composition that has declined with increasing temperatures during the viscosity measurement while heating begins to increase. When the amount of the alkaline agent is within the range of 3.5 to 7.5 parts by weight based on 100 parts by weight of the total weight of the HPMCP and the HPMC, the HPMCP may be easily solubilized, and the composition containing the alkaline agent may have an appropriate pH. In addition, the resulting capsules may have good enteric characteristics, and the separation of salt from the capsule films may be delayed during storage.

The composition for enteric hard capsules may further include a plasticizer in an amount of 1 to 15 parts by weight, for example, 5 to 10 parts by weight, based on 100 parts by weight of the total weight of the HPMCP and the HPMC. The plasticizer may be used to improve the strength of an enteric hard capsule film prepared by using the composition. Examples of the plasticizer include hydrogenated corn syrup, triethyl citrate (TEC), triacetin (TA), polyethylene glycol (PEG), propylene glycol (PG), combinations thereof. When the amount of the plasticizer is within the range of 1 to 15 parts by weight based on 100 parts by weight of the total weight of the HPMCP and the HPMC, the resulting capsules prepared by using the composition may have appropriate plasticity and have good transparency and strength.

The composition for enteric hard capsules may further include an emulsifier in an amount of 0.05 to 0.5 parts by weight, for example, 0.1 to 0.2 parts by weight, based on 100 parts by weight of the total weight of the HPMCP and the HPMC. The emulsifier may improve capsule formability. Examples of the emulsifier include sodium lauryl sulfate (SLS), sugar ester (SE), and combinations thereof. In particular, the SLS may greatly improve the capsule formability. When the amount of the emulsifier is within the range of 0.05 parts to 0.5 parts by weight based on 100 parts by weight of the total weight of the HPMCP and the HPMC, the composition may have good capsule formability, and the resulting capsules also may have good quality and good safety in regards to gastroenteric disorders when dosed.

The composition for enteric hard capsules may further include water. In this case, at least one of the HPMCP, the HPMC, the water-soluble divalent base, the alkaline agent, the plasticizer, and the emulsifier exists dissolved in water of the composition. A ratio (Ww/Wm) of the amount of water (Ww) to the total amount of the HPMCP and the HPMC (Wm) may be in the range of 75 to 85 parts by weight/15 to 25 parts by weight.

According to another embodiment of the present invention, there is provided an enteric hard capsule prepared using the composition described above.

Hereinafter, a method of preparing an enteric hard capsule by using the composition, according to an embodiment of the present invention, will be described in detail. The method according to the present embodiment of the present invention may include the following steps:
A first step is to prepare a composition for enteric hard capsules by adding the HPMCP, the HPMC, the water-soluble divalent base, and the alkaline agent to water at room temperature (for example, 20°C to 30°C). As used herein, the term "composition" indicates a composition in which at least one of the HPMCP, the HPMC, the water-soluble divalent base, and the alkaline agent is at least partially dissolved in water or at least partially gelled. In this regard, the viscosity of the composition may be in the range of 1,000 cps to 3,000 cps at room temperature. The composition prepared as described above may have a pH from 4.5 to 6.5, and a viscosity from 1,000 cps to 3,000 cps, for example, from 1,500 cps to 2,500 cps, at room temperature. The gelation start temperature of the composition may vary depending on a mixing ratio of the HPMCP, the HPMC, the water-soluble divalent base, and the alkaline agent. For example, the gelation start temperature of the composition may be from 40°C to 60°C. In this case, the composition may further include at least one of titanium dioxide and/or other colorants, such as a mineral pigment, a natural pigment, and a tar pigment.
A second step is to heat the composition to a first temperature (i.e., gelation temperature) that is higher than the gelation start temperature thereof. The first temperature may be higher than the gelation start temperature of the composition by 1°C to 25°C, for example, by 15°C to 25°C.
A third step is to cool the heated composition to a second temperature (i.e., an immersible temperature) that is lower than the gelation start temperature of the composition. The second temperature may be lower than the gelation start temperature of the composition by 15°C to 40°C, for example, by 15°C to 35°C.
A fourth step is to immerse a mold pin heated to a third temperature that is higher than the gelation start temperature into the composition. The third temperature may be higher than the gelation start temperature of the composition by 10°C to 40°C.
A fifth step is to take the mold pin out of the composition to obtain a film coated on the mold pin.
A sixth step is to maintain the film at a fourth temperature that is equal to or greater than the gelation start temperature for a first time period to fix the film onto the mold pin. The fourth temperature may be in the range of 60°C to 80°C, and the first time period may be in the range of 1 to 15 minutes (e.g., 8 minutes).
A seventh step is to dry the fixed film at a fifth temperature for a second time period to obtain a capsule shell. The fifth temperature may be in the range of 20°C to 40°C, and the second time period may be in the range of 30 to 60 minutes.

One or more embodiments of the present invention will now be described in further detail with reference to the following examples. These examples are provided for illustrative purposes only and are not intended to limit the scope of the invention.

### Examples

### Examples 1 to 9 and Comparative Examples 1 to 3

### (Preparation of Compositions)

HPMCP, HPMC, a water-soluble divalent base, an alkaline agent, a plasticizer, an emulsifier, and water were mixed in appropriate ratios as shown in Table 1 to prepare compositions for enteric hard capsules. The compositions were maintained at a temperature of 20°C.

**<Table 1>**

| | Compositions (parts by weight)^{*1} | | | | | |
|---|---|---|---|---|---|---|
| | HPMCP | HPMC | Water-soluble divalent base | Alkaline agent | Plasticizer | Emulsifier |
| | HP-50^{*2} | HPMC 2906^{*3} | Ca(OH)₂ | NaOH | PG | SLS |
| Example 1 | 80 | 20 | 2.5 | 4.0 | 5.0 | 0.1 |
| Example 2 | 80 | 20 | 2.5 | 3.5 | 5.0 | 0.1 |
| Example 3 | 80 | 20 | 2.0 | 4.0 | 5.0 | 0.1 |
| Example 4 | 80 | 20 | 2.0 | 4.5 | 5.0 | 0.1 |
| Example 5 | 80 | 20 | 1.5 | 4.5 | 5.0 | 0.1 |
| Example 6 | 80 | 20 | 1.5 | 5.0 | 5.0 | 0.1 |
| Example 7 | 80 | 20 | 1.0 | 5.5 | 5.0 | 0.1 |
| Example 8 | 80 | 20 | 1.0 | 6.0 | 5.0 | 0.1 |
| Example 9 | 80 | 20 | 0.5 | 6.0 | 5.0 | 0 |
| Comparative Example 1 | 80 | 20 | 2.6 | 6.0 | 5.0 | 0.1 |
| Comparative Example 2 | 80 | 20 | 3.0 | 4.0 | 5.0 | 0.1 |
| Comparative Example 3 | 80 | 20 | 0.05 | 7.0 | 5.0 | 0.1 |

| | | | | | | |
|---|---|---|---|---|---|---|
| * 1: In the composition, the amount of water is 4 times greater than the total weight of the HPMCP and the HPMC. * 2: HPMCP HP-50 (containing 22.3 wt% of a methoxy group, 8.5 wt% of a hydroxypropoxy group, and 25.21 wt% of a phthalyl group; and having a kinetic viscosity of 54.3 cSt), produced by Samsung Fine Chemicals Co., Ltd. * 3: AnyCoat-C BN4, produced by Samsung Fine Chemicals Co., Ltd. | | | | | | |

### (Preparation of Enteric Capsules)

Enteric capsules were prepared using each of the compositions prepared above according to the following method and the conditions shown in Table 2 below.

First, each composition was heated to a gelation temperature thereof. Then, the composition was cooled to a temperature (immersible temperature) that is lower than the gelation start temperature of the composition. Then, a mold pin (available from TECHNOPHAR, pin, #0) preheated to a temperature (mold pin temperature) that is higher than the gelation start temperature of the corresponding composition was immersed into the composition so that the mold pin was coated with the composition. After the coating process, the composition coated on the mold pin was at least partially gelated. Afterwards, the mold pin coated with the composition was maintained at a temperature of 70°C for 5 minutes and was then dried at a temperature of 30°C for 45 minutes.

**<Table 2>**

| | Gel Forming Conditions *1 | | | |
|---|---|---|---|---|
| | Gelation Start Temperature (°C) | Gelation Temperature(°C) | Immersible temperature (°C) | Mold Pin Temperature (°C) |
| Example 1 | 44.0 | 65 | 25 | 80 |
| Example 2 | 46.0 | 65 | 26 | 80 |
| Example 3 | 46.0 | 65 | 27 | 80 |
| Example 4 | 43.0 | 65 | 25 | 80 |
| Example 5 | 46.5 | 65 | 26 | 80 |
| Example 6 | 46.0 | 65 | 27 | 80 |
| Example 7 | 47.0 | 65 | 26 | 80 |
| Example 8 | 46.0 | 65 | 26 | 80 |
| Example 9 | 47.0 | 65 | 28 | 80 |
| Comparative Example 1 | 43.0 | 65 | 24 | 80 |
| Comparative Example 2 | 41.0 | 65 | 24 | 80 |
| Comparative Example 3 | 46.0 | 65 | 26 | 80 |

| | | | | |
|---|---|---|---|---|
| * 1: In Examples 1 to 9, capsules were prepared using a hot-pin process, which is a kind of thermal gelation. | | | | |

### Evaluation Example 1

Performances of capsules prepared according to Examples 1 to 9 and Comparative Examples 1 to 3 were evaluated as described below. The results are shown in Table 3 below.

### <Impact Test>

20 capsules from each example underwent impact by dropping in free fall a 70 g weight poise from a 10 cm height. Then, the number of broken capsules among the 20 tested capsules was counted. The tested capsules had a moisture content of 8±0.5 wt%.

### <Salt Separation Test>

Five capsules from each example were put in a plastic bottle, and the plastic bottles were plugged and maintained at a temperature of 20°C to 30°C and a relative humidity of 20% to 75%. Then, the appearances of the capsules were observed once a month over 12 months. The months from when the test began to when salt began to separate from the capsules were recorded.

### <Transparency>

While each dried-out capsule was held against fluorescent light, turbidity of a capsule was graded by visual inspection to one of three following categories:
⊚: clear
○: slightly unclear (if capsule surface appears slightly rough or if undissolved impurities are seen)
Δ: hazy

### Evaluation Example 2

A disintegration test was performed according to the Korean Pharmacopoeia IX. Capsules prepared according to Examples 1 to 9 and Comparative Examples 1 to 3 were immersed in a test solution at a pH of 6.8, similar to the pH of small intestinal juice, to measure disintegration time. The results are shown in Table 3.

**<Table 3>**

| | Impact test | Time when salt separation occurred (months) | Transparency | Disintegration Test | |
|---|---|---|---|---|---|
| | | | | Disintegration Time (min) | |
| | | | | pH 1.2 | pH 6.8 |
| Example 1 | 4 | Not occurred | ○ | > 120 | 3.25 |
| Example 2 | 4 | Not occurred | ○ | > 120 | 4.32 |
| Example 3 | 2 | Not occurred | ⊚ | > 120 | 3.56 |
| Example 4 | 2 | Not occurred | ⊚ | > 120 | 2.65 |
| Example 5 | 0 | Not occurred | ⊚ | > 120 | 3.04 |
| Example 6 | 0 | Not occurred | ⊚ | > 120 | 3.36 |
| Example 7 | 0 | Not occurred | ⊚ | > 120 | 3.45 |
| Example 8 | 0 | Not occurred | ⊚ | > 120 | 3.18 |
| Example 9 | 0 | Not occurred | ⊚ | > 120 | 3.46 |
| Comparative Example 1 | 6 | Not occurred | Δ | > 120 | 3.49 |
| Comparative Example 2 | 8 | Not occurred | Δ | > 120 | 3.22 |
| Comparative Example 3 | 0 | 8 months | ○ | > 120 | 4.19 |

Referring to Table 3, as compared to the capsules of Comparative Examples 1 and 2, the capsules of Examples 1 to 9 exhibit similar delaying effects of separation of salt, but have higher transparency and higher impact strength. As compared to the capsule of Comparative Example 3, the capsules of Examples 1 to 9 exhibit higher delaying effects of separation of salt and have similar or higher impact strength and transparency. The capsules of Examples 1 to 9 and Comparative Examples 1 to 3 do not disintegrate for at least 2 hours in the gastric juice conditions, but disintegrate within 5 minutes in the small intestinal juice condition, which indicates that the capsules of Examples 1 to 9 and Comparative Examples 1 to 3 all have enteric characteristics.

As described above, according to the one or more embodiments of the present invention, enteric hard capsules have similar or higher transparency and impact strength and similar or higher delaying effects of separation of salt to those of or than those of conventional capsules.

While the present invention has been particularly shown and described with reference to exemplary embodiments thereof, it will be understood by those of ordinary skill in the art that various changes in form and details may be made therein without departing from the scope of the present invention as defined by the following claims.

## Claims

1. A composition for enteric hard capsules, the composition comprising:
50 to 90 parts by weight of hydroxypropyl methylcellulose phthalate (HPMCP);
10 to 50 parts by weight of hydroxypropyl methylcellulose (HPMC);
0.1 to 2.5 parts by weight of a water-soluble divalent base; and
3.5 to 7.5 parts by weight of an alkaline agent,
wherein the amounts of the HPMCP, the HPMC, the water-soluble divalent base, and the alkaline agent in parts by weight are based on 100 parts by weight of the total weight of the HPMCP and the HPMC.

2. The composition of claim 1, wherein the HPMCP comprises 20 to 24 wt% of a methoxy group, 6 to 10 wt% of a hydroxypropoxy group, and 21 to 26 wt% of a phthalyl group, and has a kinetic viscosity of 48 cSt to 66 cSt.

3. The composition of claim 1, wherein the water-soluble divalent base comprises calcium hydroxide.

4. The composition of claim 1, wherein the alkaline agent comprises at least one selected from the group consisting of ammonia, sodium hydroxide, and potassium hydroxide.

5. The composition of claim 1, further comprising 1 to 15 parts by weight of a plasticizer based on 100 parts by weight of the total weight of the HPMCP and the HPMC.

6. The composition of claim 5, wherein the plasticizer comprises at least one selected from the group consisting of hydrogenated corn syrup, triethyl citrate (TEC), triacetin (TA), polyethylene glycol (PEG), and propylene glycol (PG).

7. The composition of claim 1, further comprising 0.05 to 0.5 parts by weight of an emulsifier based on 100 parts by weight of the total weight of the HPMCP and the HPMC.

8. The composition of claim 7, wherein the emulsifier comprises at least one selected from the group consisting of sodium lauryl sulfate (SLS) and sugar ester (SE).

9. The composition of claim 1, further comprising water, wherein a ratio (Ww/Wm) of the amount of water (Ww) to the total amount of the HPMCP and the HPMC (Wm) may be in a range of 75 to 85 parts by wight/15 to 25 parts by weight.

10. An enteric hard capsule prepared using the composition according to claim 1.
